(19) Europäisches Patentamt
European Patent Office
Office européen des brevets

(11) Publication number: 0 288 053 B1

(12) # EUROPEAN PATENT SPECIFICATION

(45) Date of publication of patent specification: **11.09.91 Bulletin 91/37**

(51) Int. Cl.$^5$: **C07J 41/00,** A61K 31/565

(21) Application number: **88106397.8**

(22) Date of filing: **21.04.88**

(54) **C17-20 lyase inhibitors.**

The file contains technical information submitted after the application was filed and not included in this specification

(30) Priority: **22.04.87 US 41172**

(43) Date of publication of application: **26.10.88 Bulletin 88/43**

(45) Publication of the grant of the patent: **11.09.91 Bulletin 91/37**

(84) Designated Contracting States: **AT BE CH DE ES FR GB GR IT LI LU NL SE**

(56) References cited:
**FR-A- 1 432 523**
**B.J. Taylor; The design, synthesis and characterization of site- specific inhibitors of Steroidogenic cytochrome P450 enzymes MIT (1983)-thesis**

(73) Proprietor: **MERRELL DOW PHARMACEUTICALS INC.**
**2110 East Galbraith Road**
**Cincinnati Ohio 45215-6300 (US)**

(72) Inventor: **Angelastro, Michael R.**
**3018 Strasford Court**
**Loveland Ohio 45140 (US)**
Inventor: **Blohm, Thomas R.**
**6468 Barn Court**
**Cincinnati Ohio 45243 (US)**

(74) Representative: **Vossius & Partner**
**Siebertstrasse 4 P.O. Box 86 07 67**
**W-8000 München 86 (DE)**

EP 0 288 053 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to a group of compounds having the following general formula :

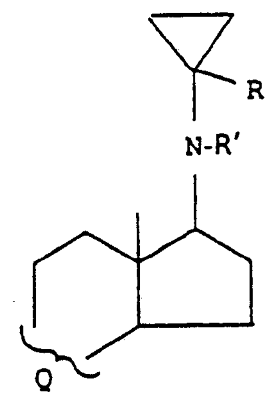

wherein R is hydrogen or methyl ; R' is hydrogen, $C_1$-$C_4$ alkyl or cyclopropyl ; and Q is

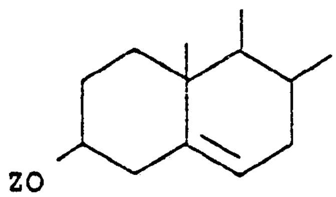

or

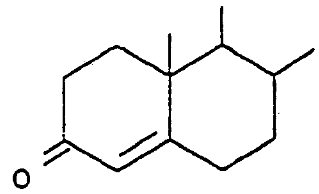

I II

wherein Z is hydrogen, alkanoyl of 1-10 carbon atoms or cyclopentane- and benzene-alkanoyl wherein the alkanoyl portion contains up to 4 carbon atoms, except the compound 17β-(cyclopropylamino)-androst-5-en-3β-ol. Examples of alkanoyl group are acetyl, propionyl, butanoyl, and decanoyl ; examples of the cyclopentane- and benzenealkanoyl groups are cyclopentanepropionyl and benzenepropionyl. Preferred compounds are those in which Q is structure I.

Acid addition salts of the aforesaid compounds with pharmaceutically acceptable acids are equivalent to the above amines for the purposes of this invention. Illustrative of such salts are the salts with inorganic acids such as, for example, hydrochloric, hydrobromic, sulfuric, phosphoric and like acids ; with organic carboxylic acids such as, for examples, acetic, propionic, glycolic, lactic, pyruvic, malonic, succinic, fumaric, malic, tartaric, citric, ascorbic, maleic, hydroxymaleic and dihydroxymaleic, benzoic, phenylacetic, 4-aminobenzoic, 4-hydroxybenzoic, anthranilic, cinnamic, salicylic, 4-aminosalicylic, 2-phenoxybenzoic, 2-acetoxybenzoic, mandelic and like acids ; and with organic sulfonic acids such as methanesulfonic acid and p-toluenesulfonic acids.

The compounds of the present invention are conveniently prepared by the reduction of an appropriate steroidal imine or enamine, wherein the compound used is a 3-hydroxy or 3-alkanoylhydroxy-$\Delta^5$-steroid, with a hydride reducing agent. Where the starting material is an imine, the reaction can be illustrated as follows :

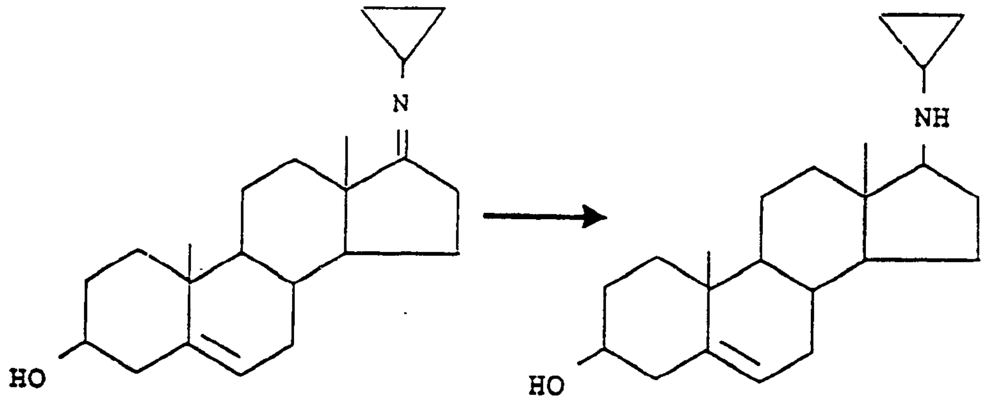

In this case, the reduction is carried out using a hydride reducing agent, preferably sodium borohydride, in an alkanol solvent. Prior to carrying out the reduction shown above, the steroid starting material can be acylated using, for example, acetic anhydride to give the corresponding 3-acetoxy steroid which is then reduced with borohydride as described earlier to give the corresponding 3-acetoxy product. In either case, the product obtained is a secondary amine which can be converted to the corresponding N-methyl compound by treatment with formaldehyde and formic acid in an Eschweiler-Clarke reaction or by reaction with aqueous formaldehyde and sodium borohydride.

Those compounds wherein R' is $C_{2-4}$ alkyl can be obtained from a 17-cyclopropylamino steroid. This is reacted, for example, with acetyl chloride to give the corresponding acetamide which is then reduced with sodium cyanoborohydride to give the N-ethyl compound. In those cases where the above reaction with acetyl chloride also gives the 3-ester, the ester group can be removed, after the reduction, by base hydrolysis such as a combination of potassium carbonate, methanol and tetrahydrofuran or by acid hydrolysis such as with hydrochloric acid. In the latter case, the hydrochloride salt is obtained.

The 3-hydroxy-$\Delta^5$-compound obtained above can optionally be converted to the corresponding 3-keto-$\Delta^4$-compound by an Oppenauer oxidation using aluminum isopropoxide.

The imine starting material used in this process can be obtained by the reaction of dehydroepiandrosterone with the appropriate cyclopropylamine in refluxing methanol. The reaction is carried out in the presence of a dehydrating agent to remove water from the reaction mixture as it is formed.

When the reduction referred to initially is carried out on an enamine, borane or a borohydride reducing agent such as sodium borohydride is used as the reducing agent. The necessary enamine starting material is obtained by the condensation of dehydroepiandrosterone with an appropriate secondary amine such as dicyclopropylamine. The alcohol final product obtained in this process can be acylated with an appropriate anhydride, such as acetic anhydride, to give the corresponding 3-acetoxy compound or it can be oxidized in an Oppenauer oxidation to give the corresponding 3-keto-$\Delta^4$-compound.

The present compounds are useful as inhibitors of steroid $C_{17-20}$ lyase and thus inhibit testosterone formation. Consequently, they are useful for treating various androgen-dependent disorders. More particularly, the present compounds are useful in the treatment of prostatic carcinoma, benign prostatic hyperplasia and virilism and hirsutism (in women).

It is well established that reduction of serum testosterone levels is useful in the treatment of many cases of prostatic carcinoma. In clinical practice, this has been accomplished by orchiectomy or by diethylstilbestrol treatment but the first approach is often psychologically unacceptable while a number of side effects are associated with the second approach. Thus, an alternative approach to testosterone reduction is desirable and this can be accomplished by the administration of the present compounds. To the extent that prostatic carcinoma is androgen-dependent, the present compounds would block the source of androgens and thus serve as an appropriate treatment for this condition.

The activity of the present compounds as inhibitors of steroid $C_{17-20}$ lyase was established using microsomal preparations of the steroid $C_{17-20}$ lyase enzyme from human or laboratory animal testis ; human testes used for this purpose were obtained from therapeutic orchiectomies. The enzyme was incubated with NADPH and the test compound in the concentration range $5 \times 10^{-8}$M to $3 \times 10^{-6}$M and the extent of inhibition of the enzyme was determined with time-dependency of inhibition being established by a decline in enzyme activity with the time of exposure to the test compound. Time-dependency of inhibition often implies irreversible inactivation of the enzyme and irreversibility was specifically established by inability to restore enzyme activity by dialysis under conditions which maintained activity of native enzyme. When tested according to the above procedure using human enzyme, the compounds of the present invention were found to inhibit the enzyme in a time-dependent manner and irreversibly.

In the treatment of the various androgen-dependent disorders described earlier, the compounds of the present invention may be administered orally to the patient being treated to achieve the particular effect desired. The amount of compound to be administered will vary over a wide range and can be any effective amount. Depending on the patient to be treated, and the severity of the condition being treated, the effective amount of compound administered will vary from about 0.625 to 62.5 mg/kg of body weight per day and preferably from 5 to 30 mg/kg of body weight per day. Unit dosages for oral administration may contain, for example, from 25 to 500 mg of a compound of the invention. Alternatively, the present compounds can be administered by parenteral routes or by implants.

In practicing the method of this invention, the active ingredient is preferably incorporated in a composition containing a pharmaceutical carrier and from about 5 to about 90% by weight of the cyclopropylamino steroid or a pharmaceutically-acceptable salt thereof. The term "pharmaceutical carrier" refers to known pharmaceuticals excipients useful in formulating pharmaceutically active compounds for internal administration to animals, and which are substantially non-toxic and non-sensitizing under conditions of use. The compositions can be prepared by known techniques for the preparation of tablets or capsules and can contain suitable excipients known to be useful in the preparation of the particular type of composition desired. Suitable pharmaceutical carriers in formulation techniques are found in standard texts, such as Remingtons Pharmaceutical Sciences, Mack Publishing Company, Easton, Pennsylvania.

The following examples are presented to illustrate the present invention but they should not be construed as limiting it in any way.

EXAMPLE 1

To a solution of 21 g of dehydroepiandrosterone in a mixture of 175 ml of cyclopropylamine and 150 ml of methanol there was added 5 g of molecular sieves. The reaction mixture was refluxed for 48 hours, cooled to room temperature, and filtered through magnesium sulfate. The magnesium sulfate was washed with ethyl acetate and the solvent was removed from the combined filtrates under reduced pressure to give 17-(cyclopropylimino)androst-5-en-3β-ol melting at about 187°C.

EXAMPLE 2 (the compound described is not claimed)

To a solution of 9.1 g of 17-(cyclopropylimino)androst-5-en-3β-ol in 200 ml of dry ethanol was added 2 g of sodium borohydride. The reaction mixture was stirred at room temperature for 3 hours and then 100 ml of solvent was removed from the mixture under reduced pressure. The reaction mixture was then quenched with dilute acetic acid, diluted with 600 ml of water, and the pH was adjusted to 14 by the addition of sodium hydroxide. The aqueous mixture was extracted 3 times with 600 ml potions of ethyl acetate and the combined organic extracts were dried over magnesium sulfate. Removal of the solvent under reduced pressure gave the desired 17β-(cyclopropylamino)androst-5-en-3β-ol as a while solid ; MS (m/z) : 370 $(M + 41)^+$, 358 $(M + 29)^+$, 330$(M + H)^+$, 312 $(MH\text{-}H_2O)^+$. This compound has the following structural formula :

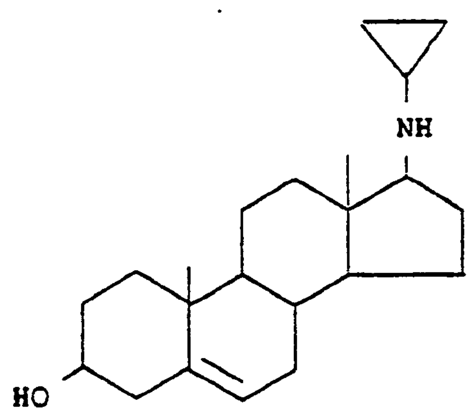

This compound is already known from the thesis of B.J. TAYLOR entitled "The design, synthesis and characterization of site-specific inhibitors of steroidogenic cytochrome P 450 enzymes" MIT (1983).

EXAMPLE 3

Reaction of dehydroepiandrosterone with 1-methylcyclopropylamine according to the procedure described in Example 1 gives 17-(1-methylcyclopropylimino)androst-5-en-3β-ol. This is then reduced with sodium borohydride according to the procedure in Example 2 to give 17β-(1-methylcyclopropylamino)androst-5-en-3β-ol.

EXAMPLE 4

Reaction of 17-(cyclopropylimino)androst-5-en-3β-ol with an excess of acetic anhydride in the presence of a base (pyridine) followed by removal of the excess anhydride and acetic acid gives 3β-acetoxy-17-(cyclopropylimino)androst-5-ene. Reduction of this ester with sodium borohydride according to the procedure described in Example 2 gives 3β-acetoxy-17β-(cyclopropylamino)androst-5-ene. 3β-(Cyclopentanepropionyloxy)-17β-(cyclopropylamino)androst-5-ene and 3β-(benzenepropionyloxy)-17β-(cyclopropylamino)androst-5-ene are obtained in a similar way using the appropriate acid chlorides.

EXAMPLE 5

To a mixture of 10 ml of formic acid and 5 ml of formaldehyde was added 1.4 g of 17β-(cyclopropylamino)androst-5-en-3β-ol. The mixture was heated at reflux for 5 hours, the volume was then reduced to 7.5 ml *in vacuo*, and 10 ml of 50% (w/w) aqueous sodium hydroxide was added. The aqueous layer was separated and extracted with ethyl acetate and the combined organic solutions were dried over magnesium sulfate. The solvent was then removed *in vacuo* and the residual product was purified by flash chromatography to give 17β-[N-methyl(cyclopropylamino)]androst-5-en-3β-ol.

EXAMPLE 6

A solution of 1.5 grams of 17β-(cyclopropylamino)androst-5-en-3β-ol in 80 ml of toluene was concentrated to 75% of its original volume and 20 ml of cyclohexanone was added. The mixture was again concentrated to 75% of its volume and 1.5 g of aluminum isopropoxide was added. The reaction mixture was refluxed for 45 minutes, cooled to room temperature, and 50 ml of water and 5 ml of concentrated hydrochloric acid were added. The solution was then treated with 11 g of sodium hydroxide and the two phases were separated. The aqueous phase was extracted with 50 ml of ethyl acetate and the combined organic extracts were dried over sodium sulfate. The solvent was removed *in vacuo* and crystallization of the residue from hexane/ethyl acetate gave 17β-(cyclopropylamino)androst-4-en-3-one. MS (m/z) : 327 ($M^+$), 312 ($M^+ - CH_3$).

## Claims

### Claims for the following Contracting States : AT BE CH DE FR GB IT LI LU NL SE

1. A compound of the formula

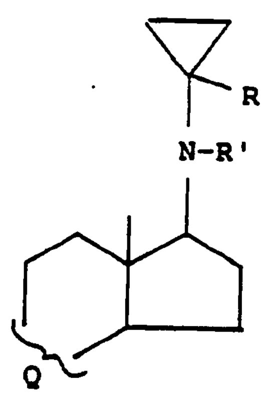

wherein R is hydrogen or methyl ; R′ is hydrogen, $C_1$-$C_4$ alkyl or cyclopropyl ; and Q is

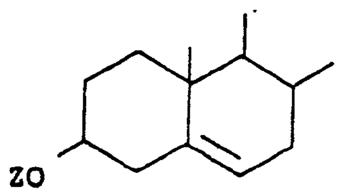

or

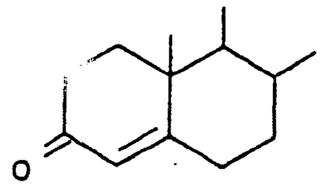

wherein Z is hydrogen or alkanoyl of 1-10 carbon atoms, or cyclopentane- or benzene-alkanoyl wherein the alkanoyl portion contains up to 4 carbon atoms, except the compound 17β-(cyclopropylamino) androst-5-en-3β-ol.

2. A compound according to Claim 1 which has the formula :

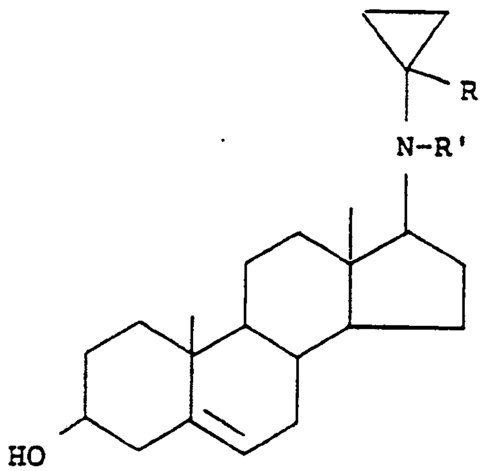

wherein R is hydrogen or methyl ; and R′ is hydrogen, $C_1$-$C_4$ alkyl or cyclopropyl.

3. A compound according to Claim 1 which has the formula :

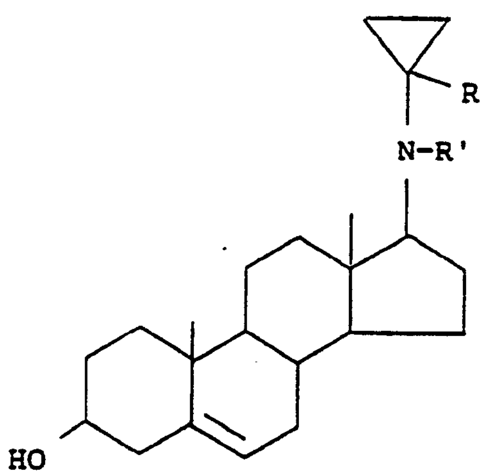

wherein R is hydrogen or methyl ; and R′ is hydrogen or $C_{1-4}$ alkyl.

4. A compound according to Claim 1 which is 17β-[N-methyl(cyclopropylamino)]androst-5-en-3β-ol.

5. A compound according to Claim 1 which is 17β-(cyclopropylamino)androst-4-en-3-one.

6. Use of a compound according to any of claims 1 to 5 for the preparation of pharmaceutical drugs for treating androgen-dependent disorders.

7. Pharmaceutical preparations for treating androgen-dependent disorders comprising a pharmaceutical effective amount of a compound according to any of claims 1 to 5 and optionally usual additives and/or carriers.

**Claims for the following Contracting State : GR**

1. A compound of the formula

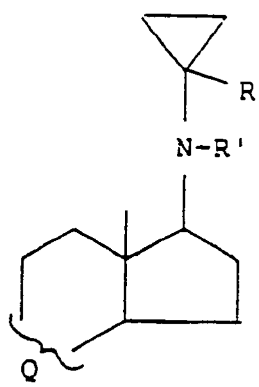

wherein R is hydrogen or methyl ; R′ is hydrogen, $C_1$-$C_4$ alkyl or cyclopropyl ; and Q is

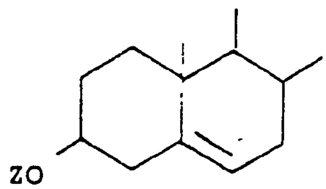

or

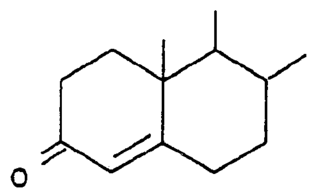

wherein Z is hydrogen or alkanoyl of 1-10 carbon atoms, or cyclopentane- or benzene-alkanoyl wherein the alkanoyl portion contains up to 4 carbon atoms, except the compound 17β-(cyclopropylamino)androst-5-en-3β-ol.

2. A compound according to Claim 1 which has the formula :

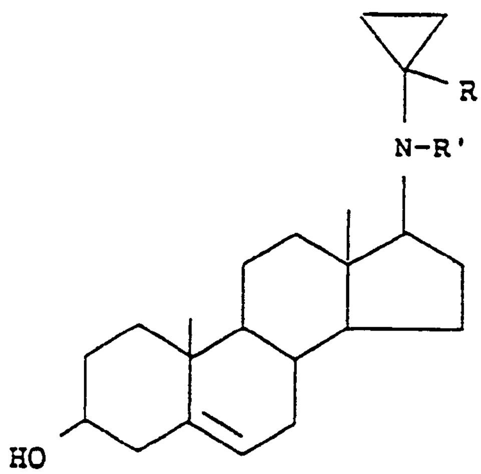

wherein R is hydrogen or methyl ; and R′ is hydrogen, $C_1$-$C_4$ alkyl or cyclopropyl.

3. A compound according to Claim 1 which has the formula :

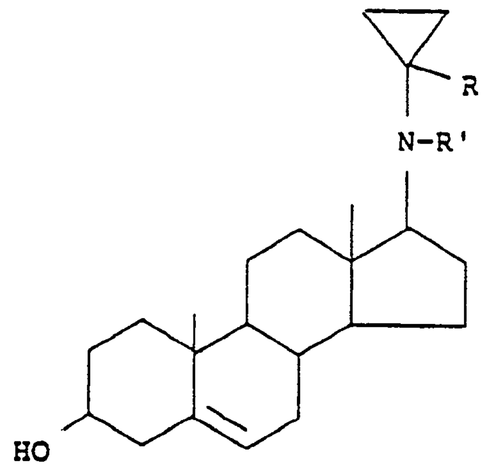

wherein R is hydrogen or methyl ; and R' is hydrogen or $C_{1-4}$ alkyl.

4. A compound according to Claim 1 which is 17β-(N-methyl(cyclopropylamino)]androst-5-en-3β-ol.

5. A compound according to Claim 1 which is 17β-(cyclopropylamino)androst-4-en-3-one.

6. Use of a compound according to any of claims 1 to 5 for the preparation of pharmaceutical drugs for treating androgen-dependent disorders.

**Claims for the following Contracting State : ES**

1. A process for preparing a compound of the formula

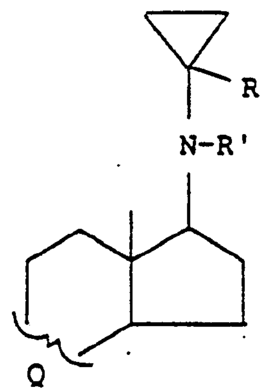

wherein R is hydrogen or methyl ; R' is hydrogen, $C_1$-$C_4$ alkyl or cyclopropyl ; and Q is

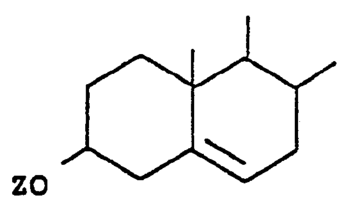

or

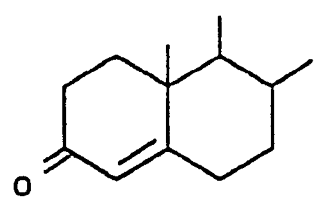

wherein Z is hydrogen or alkanoyl of 1-10 carbon atoms, or cyclopentane- or benzene-alkanoyl wherein the alkanoyl portion contains up to 4 carbon atoms, which comprises reacting an amine of the formula

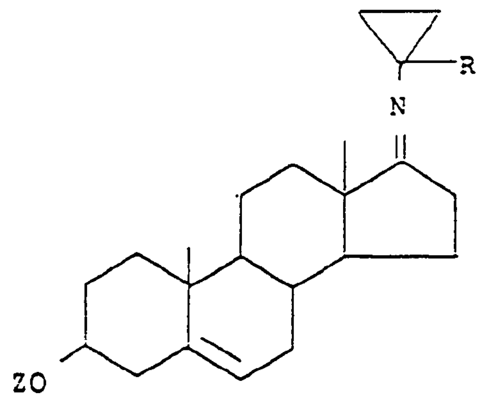

wherein R and Z are defined as above, with a hydride reducing agent optionally followed by :

(a) Reaction with formaldehyde and formic acid to give the compound in which R′ is methyl ; or

(b) Reaction with a $C_{2-4}$ alkanoyl chloride to give the corresponding alkanamide followed by reduction with sodium cyanoborohydride to give the compounds in which R′ is $C_{2-4}$ alkyl ; or

(c) Oppenauer oxidation with aluminum isopropoxide to give the corresponding 3-keto-$\Delta^4$-compound.

2. A process according to Claim 1 for preparing a compound of the formula :

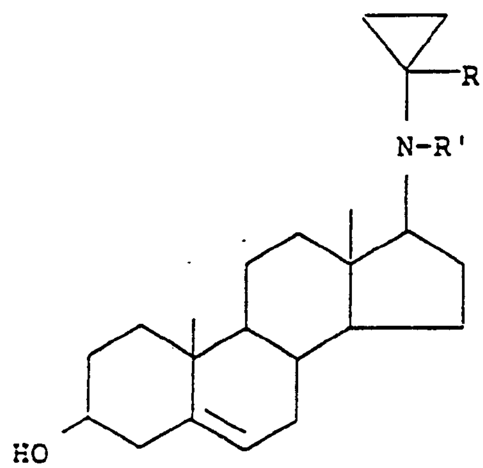

wherein R is hydrogen or methyl ; and R′ is hydrogen or $C_1$-$C_4$ alkyl which comprises reacting an amine of the formula

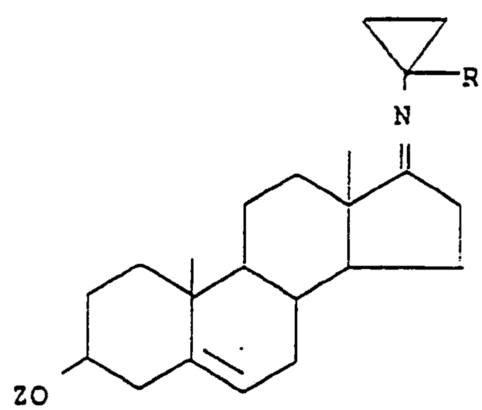

wherein R and Z are defined as above, with a hydride reducing agent optionally followed by :

(a) Reaction with formaldehyde and formic acid to give the compound in which R′ is methyl ; or

(b) Reaction with a $C_{2-4}$ alkanoyl chloride to give the corresponding alkanamide followed by reduction with sodium cyanoborohydride to give the compounds in which R′ is $C_{2-4}$ alkyl.

3. A process according to Claim 1 for preparing 17β-(cyclopropylamino)androst-5-en-3β-ol which comprises reacting 17-(cyclopropylimino)androst-5-en-3β-ol with sodium borohydride.

4. A process according to Claim 1 for preparing 17β-(cyclopropylamino)androst-4-en-3-one which comprises reacting 17-(cyclopropylimino)androst-5-en-3β-ol with sodium borohydride followed by oxidation with aluminum isopropoxide.

5. Use of the compounds obtainable according to any of claims 1 to 4 for the preparation of drugs for treating androgen-dependent disorders.

## Patentansprüche

### Patentansprüche für folgende Vertragsstaaten : AT BE CH DE FR GB IT LI LU NL SE

1. Verbindung der Formel

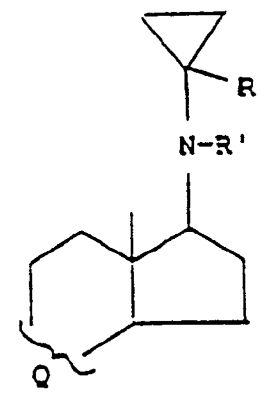

in der R ein Wasserstoffatom oder eine Methylgruppe bedeutet, R' ein Wasserstoffatom, eine $C_1$-$C_4$-Alkyl- oder Cyclopropylgruppe darstellt und Q eine Gruppe der Formel

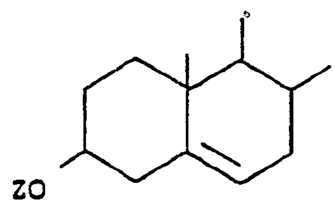

oder

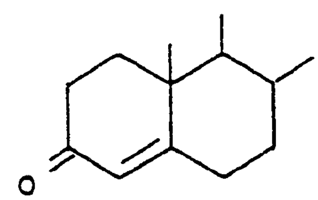

darstellt, wobei Z ein Wasserstoffatom oder einen Alkanoylrest mit 1 bis 10 Kohlenstoffatomen, oder einen Cyclopentan- oder Benzolalkanoylrest bedeutet, wobei der Alkanoyl-Anteil bis zu 4 Kohlenstoffatome enthält, ausgenommen die Verbindung 7β-(Cyclopropylamino)-androst-5-en-3β-ol.

2. Verbindung nach Anspruch 1 der Formel

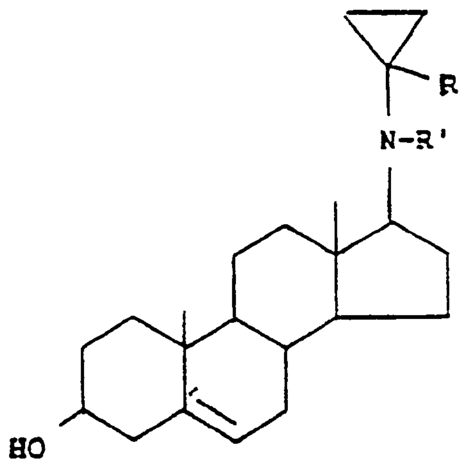

in der R ein Wasserstoffatom oder eine Methylgruppe bedeutet und R′ ein Wasserstoffatom, eine $C_1$-$C_4$-Alkyl- oder Cyclopropylgruppe darstellt.

3. Verbindung nach Anspruch 1 der Formel

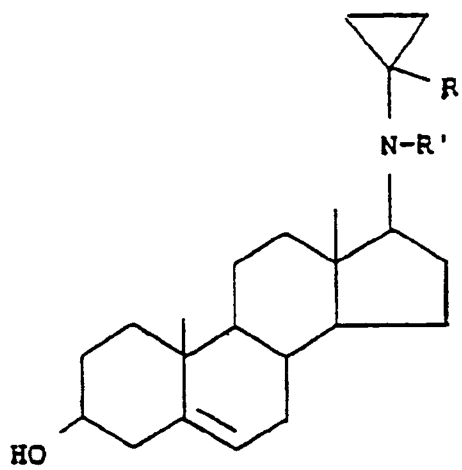

in der R ein Wasserstoffatom oder eine Methylgruppe bedeutet und R′ ein Wasserstoffatom oder einen $C_1$-$C_4$-Alkylrest darstellt.

4. Verbindung nach Anspruch 1, nämlich 17β-[N-Methyl-(cyclopropylamino]-androst-5-en-3β-ol.

5. Verbindung nach Anspruch 1, nämlich 17β-(Cyclopropylamino)-androst-4-en-3-on.

6. Verwendung einer Verbindung nach einem der Ansprüche 1 bis 5 zur Herstellung von Arzneimitteln für die Behandlung von Androgen-abhängigen Störungen.

7. Arzneimittel zur Behandlung von Androgen-abhängigen Störungen, umfassend eine pharmazeutisch wirksame Menge einer Verbindung nach einem der Ansprüche bis 5 und gegebenenfalls übliche Zusatzstoffe und/oder Träger.

**Patentansprüche für folgenden Vertragsstaat : GR**

1. Verbindung der Formel

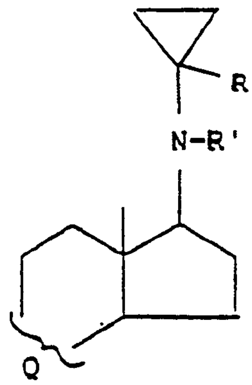

in der R ein Wasserstoffatom oder eine Methylgruppe bedeutet, R′ ein Wasserstoffatom, eine $C_1$-$C_4$-Alkyl- oder Cyclopropylgruppe darstellt und Q eine Gruppe der Formel

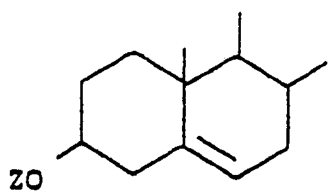

oder

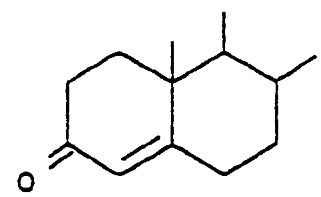

darstellt, wobei Z ein Wasserstoffatom oder einen Alkanoylrest mit 1 bis 10 Kohlenstoffatomen, oder einen Cyclopentan- oder Benzolalkanoylrest bedeutet, wobei der Alkanoyl-Anteil bis zu 4 Kohlenstoffatome enthält, ausgenommen die Verbindung 17β-(Cyclopropylamino)-androst-5-en-3β-ol.

2. Verbindung nach Anspruch 1 der Formel

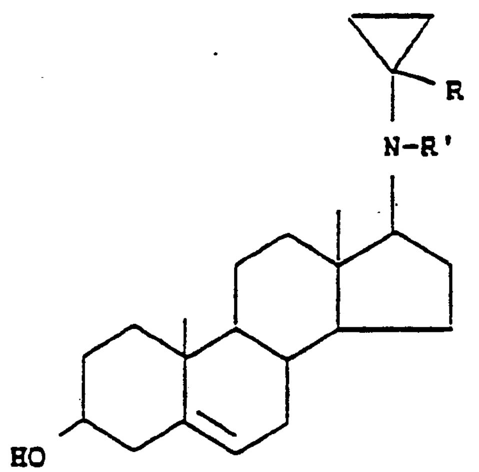

in der R ein Wasserstoffatom oder eine Methylgruppe bedeutet und R′ ein Wasserstoffatom, eine $C_1$-$C_4$-Alkyl- oder Cyclopropylgruppe darstellt.

3. Verbindung nach Anspruch 1 der Formel

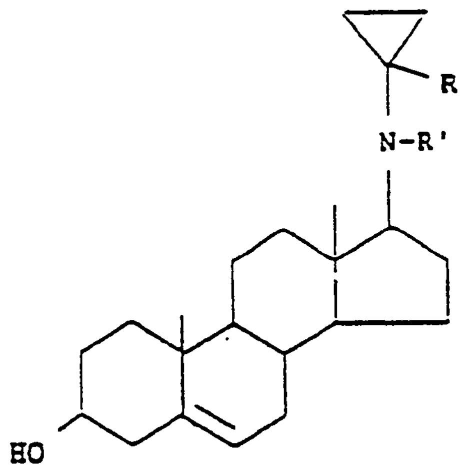

in der R ein Wasserstoffatom oder eine Methylgruppe bedeutet und R' ein Wasserstoffatom oder einen $C_1$-$C_4$-Alkylrest darstellt.

4. Verbindung nach Anspruch 1, nämlich 17β-[N-Methyl-(cyclopropylamino)]-androst-5-en-3β-ol.

5. Verbindung nach Anspruch 1, nämlich 17β-(Cyclopropylamino)-androst-4-en-3-on.

6. Verwendung einer Verbindung nach einem der Ansprüche 1 bis 5 zur Herstellung von Arzneimitteln für die Behandlung von Androgen-abhängigen Störungen.

**Patentansprüche für folgenden Vertragsstaat : ES**

1. Verfahren zur Herstellung einer Verbindung der Formel

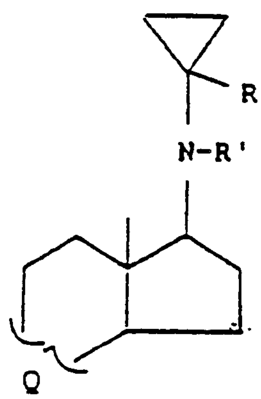

in der R ein Wasserstoffatom oder eine Methylgruppe bedeutet, R' ein Wasserstoffatom, eine $C_1$-$C_4$-Alkyl- oder Cyclopropylgruppe darstellt und Q eine Gruppe der Formel

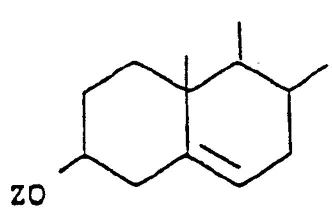

oder

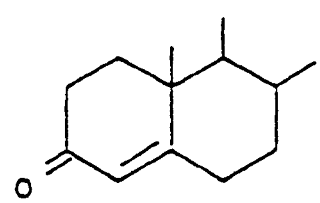

darstellt, wobei Z ein Wasserstoffatom oder einen Alkanoylrest mit 1 bis 10 Kohlenstoffatomen, oder einen Cyclopentan- oder Benzolalkanoylrest bedeutet, wobei der Alkanoyl-Anteil bis zu 4 Kohlenstoffatome enthält, umfassend die Umsetzung eines Imins der Formel

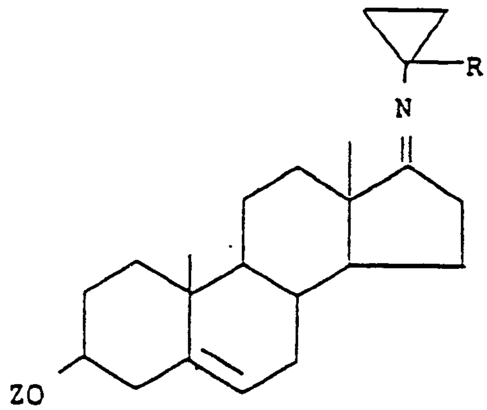

wobei R und Z wie vorstehend definiert sind, mit einem Hydrid-Reduktionsmittel, gegebenenfalls gefolgt von :

(a) Umsetzung mit Formaldehyd und Ameisensäure zu der Verbindung, in der R′ eine Methylgruppe bedeutet, oder

(b) Umsetzung mit einem $C_{2-4}$-Alkanoylchlorid zum entsprechenden Alkanamid, gefolgt von einer Reduktion mit Natrium-Cyanoborhydrid zu Verbindungen, in denen R′ einen $C_{2-4}$-Alkylrest bedeutet, oder

(c) Oppenauer-Oxidation mit Aluminiumisopropoxid zur entsprechenden 3-Keto-$\Delta^4$-Verbindung

2. Verfahren nach Anspruch 1 zur Herstellung einer Verbindung der Formel

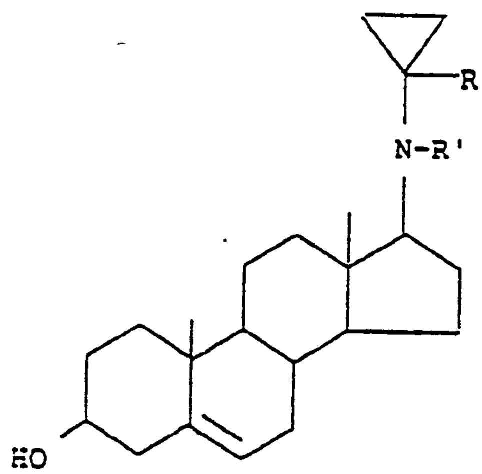

in der R ein Wasserstoffatom oder eine Methylgruppe bedeutet und R′ ein Wasserstoffatom oder einen $C_1$-$C_4$-Alkylrest darstellt, umfassend die Umsetzung eines Imins der Formel

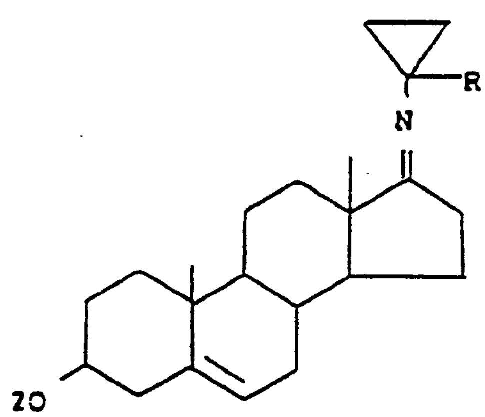

in der R und Z wie vorstehend definiert sind, mit einem Hydrid-Reduktionsmittel, gegebenenfalls gefolgt von

(a) Umsetzung mit Formaldehyd und Ameisensäure zur Verbindung, in der R′ eine Methylgruppe bedeutet,

oder

(b) Umsetzung mit einem $C_{2-4}$-Alkanoylchlorid zum entsprechenden Alkanamid, gefolgt von Reduktion mit Natrium-cyanoborhydrid zu den Verbindungen, in denen R' einen $C_{2-4}$-Alkylrest bedeutet.

3. Verfahren nach Anspruch zur Herstellung von 17β-(Cyclopropylamino)-androst-5-en-3β-ol, umfassend die Umsetzung von 17-(Cyclopropylimino)-androst-5-en-3β-ol mit Natriumborhydrid.

4. Verfahren nach Anspruch zur Herstellung von 17β-(Cyclopropylamino)-androst-4-en-3-on, umfassend die Umsetzung von 17-(Cyclopropylimino)-androst-5-en-3β-ol mit Natriumborhydrid, gefolgt von Oxidation mit Aluminium-isopropoxid.

5. Verwendung der Verbindungen, die nach einem der Ansprüche 1 bis 4 erhältlich sind, für die Herstellung von Arzneistoffen zur Behandlung von Androgen-abhängigen Störungen.

## Revendications

### Revendications pour les Etats Contractants : AT BE CH DE FR GB IT LI LU NL SE

1. Composé de formule

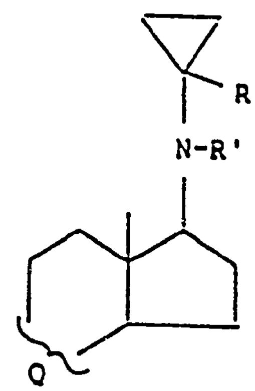

dans laquelle R est un atome d'hydrogène ou le groupe méthyle ; R' est un atome d'hydrogène, un groupe alkyle en $C_1$-$C_4$ ou cyclopropyle ; et Q est

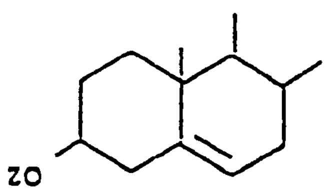

ou

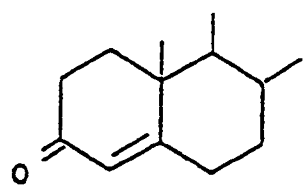

où Z est un atome d'hydrogène ou un groupe alcanoyle ayant 1-10 atomes de carbone, ou cyclopentane- ou benzène-alcanoyle où la portion alcanoyle contient jusqu'à 4 atomes de carbone, à l'exception du composé 17β-(cyclopropylamino)androst-5-èn-3β-ol.

2. Composé selon la revendication 1, qui possède la formule

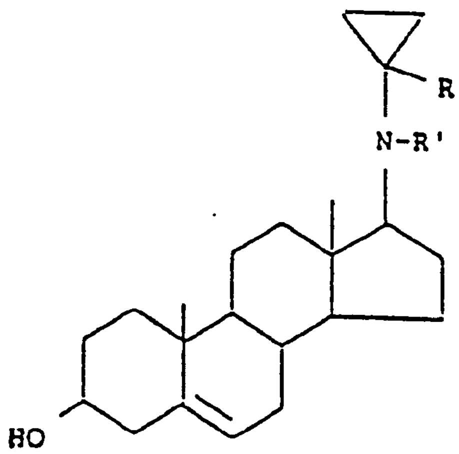

dans laquelle R est un atome d'hydrogène ou le groupe méthyle ; et R' est un atome d'hydrogène, un groupe alkyle en $C_1$-$C_4$ ou cyclopropyle.

3. Composé selon la revendication 1, qui possède la formule

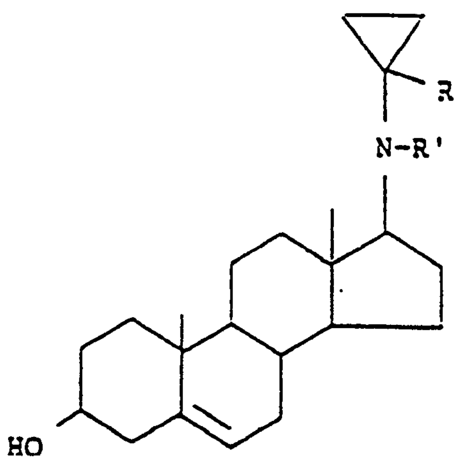

dans laquelle R est un atome d'hydrogène ou le groupe méthyle ; et R' est un atome d'hydrogène ou un groupe alkyle en $C_1$-$C_4$.

4. Composé selon la revendication 1, qui est le 17β-[N-méthyl(cyclopropylamino)]androst-5-èn-3β-ol.

5. Composé selon la revendication 1, qui est la 17β-(cyclopropylamino)androst-4-èn-3-one.

6. Utilisation d'un composé selon l'une quelconque des revendications 1 à 5, pour la préparation de médicaments pharmaceutiques pour le traitement des troubles androgéno-dépendants.

7. Préparations pharmaceutiques pour le traitement des troubles androgéno-dépendants, comprenant une quantité pharmaceutique efficace d'un composé selon l'une quelconque des revendications 1 à 5 et éventuellement des additifs et/ou des véhicules classiques.

**Revendications pour l'Etat Contractant : GR**

1. Composé de formule

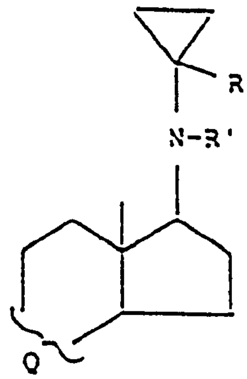

dans laquelle R est un atome d'hydrogène ou le groupe méthyle ; R′ est un atome d'hydrogène, un groupe alkyle en $C_1$-$C_4$ ou cyclopropyle ; et Q est

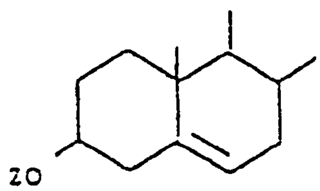

ou

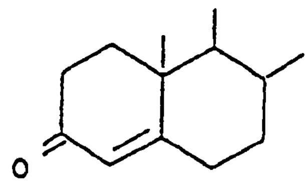

où Z est un atome d'hydrogène ou un groupe alcanoyle ayant 1-10 atomes de carbone, ou cyclopentane- ou benzène-alcanoyle où la portion alcanoyle contient jusqu'à 4 atomes de carbone, à l'exception du composé 17β-(cyclopropylamino)androst-5-èn-3β-ol.

2. Composé selon la revendication 1, qui possède la formule :

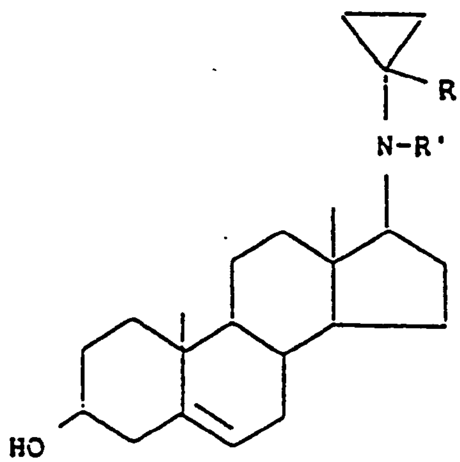

dans laquelle R est un atome d'hydrogène ou le groupe méthyle ; et R′ est un atome d'hydrogène, un groupe alkyle en $C_1$-$C_4$ ou cyclopropyle.

3. Composé selon la revendication 1, qui possède la formule

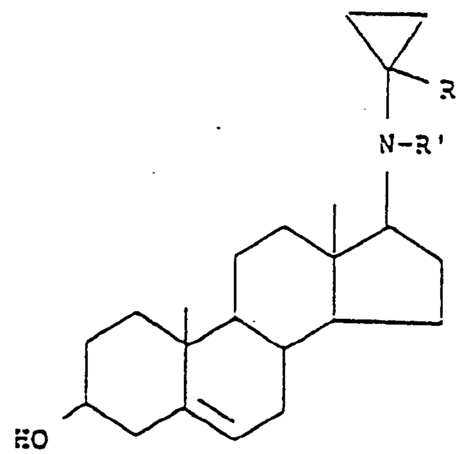

dans laquelle R est un atome d'hydrogène ou le groupe méthyle ; et R' est un atome d'hydrogène ou un groupe alkyle en $C_1$-$C_4$.

4. Composé selon la revendication 1, qui est le 17β-[(N-méthyl(cyclopropylamino)]androst-5-èn-3β-ol.

5. Composé selon la revendication 1, qui est la 17β-(cyclopropylamino)androst-4-èn-3-one.

6. Utilisation d'un composé selon l'une quelconque des revendications 1 à 5, pour la préparation de médicaments pharmaceutiques pour le traitement des troubles androgéno-dépendants.

**Revendications pour l'Etat Contractant : ES**

1. Procédé pour la préparation d'un composé de formule

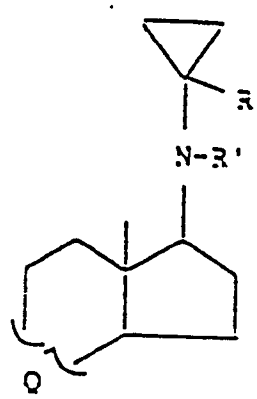

dans laquelle R est un atome d'hydrogène ou le groupe méthyle ; R' est un atome d'hydrogène, un groupe alkyle en $C_1$-$C_4$ ou cyclopropyle ; et Q est

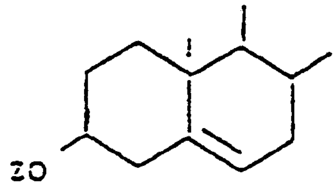

ou

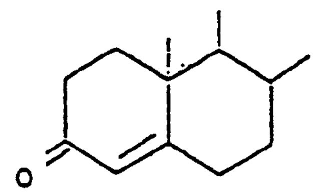

où Z est un atome d'hydrogène ou un groupe alcanoyle ayant 1-10 atomes de carbone, ou cyclopentane- ou benzène-alcanoyle où la portion alcanoyle contient jusqu'à 4 atomes de carbone, qui comprend la réaction d'une imine de formule :

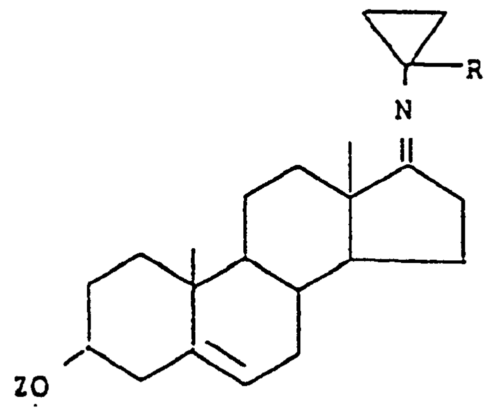

dans laquelle R et Z sont tels que définis ci-dessus, avec un agent réducteur qui est un hydrure, celle-ci étant éventuellement suivie de :

(a) la réaction avec le formaldéhyde et l'acide formique pour donner le composé dans lequel R' est le groupe méthyle ; ou

(b) la réaction avec un chlorure d'alcanoyle en $C_2$-$C_4$ pour donner l'alcanamide correspondant, suivie de la réduction avec le cyanoborohydrure de sodium pour donner les composés dans lesquels R' est un groupe alkyle en $C_2$-$C_4$ ; ou

(c) l'oxydation d'Oppenauer avec l'isopropoxyde d'aluminium pour donner le 3-céto-$\Delta^4$-composé correspondant.

2. Procédé selon la revendication 1, pour la préparation d'un composé de formule :

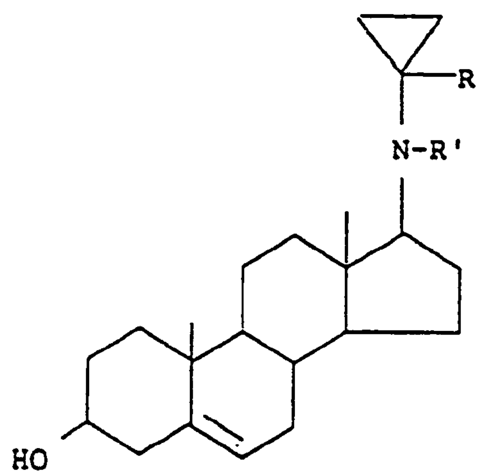

dans laquelle R est un atome d'hydrogène ou le groupe méthyle ; et R' est un atome d'hydrogène ou un groupe alkyle en $C_1$-$C_4$, qui comprend la réaction d'une imine de formule :

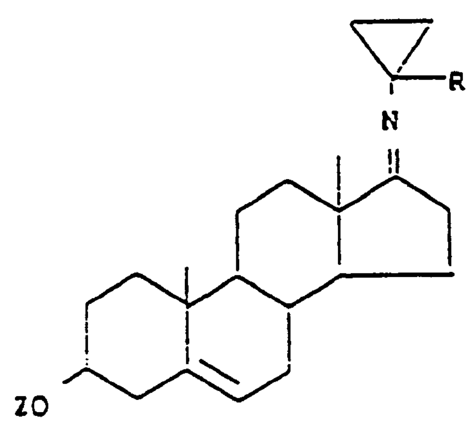

dans laquelle R et Z sont tels que définis ci-dessus, avec un agent réducteur qui est un hydrure, celle-ci étant éventuellement suivie de :

(a) la réaction avec le formaldéhyde et l'acide formique pour donner le composé dans lequel R′ est le groupe méthyle ; ou

(b) la réaction avec un chlorure d'alcanoyle en $C_2$-$C_4$ pour donner l'alcanamide correspondant, suivie de la réduction avec le cyanoborohydrure de sodium pour donner les composés dans lesquels R′ est un groupe alkyle en $C_2$-$C_4$.

3. Procédé selon la revendication 1 pour la préparation du 17β-(cyclopropylamino)androst-5-èn-3β-ol, qui comprend la réaction du 17-(cyclopropylimino)androst-5-èn-3β-ol avec le borohydrure de sodium.

4. Procédé selon la revendication 1 pour la préparation de la 17β-(cyclopropylamino)androst-4-èn-3-one, qui comprend la réaction du 17-(cyclopropylimino)androst-5-èn-3β-ol avec le borohydrure de sodium, suivie de l'oxydation avec l'isopropoxyde d'aluminium.

5. Utilisation des composés pouvant être obtenus selon l'une quel-conque des revendications 1 à 4 pour la préparation de médicaments pour le traitement des troubles androgéno-dépendants.